# EUROPEAN PATENT SPECIFICATION

(11) **EP 2 838 545 B1**
(45) Date of publication and mention of the grant of the patent: **04.12.2019**
(21) Application number: 13764190.8
(22) Date of filing: 25.03.2013
(51) Int. Cl.: A61K 36/882, A61K 36/236, A61K 36/232, A61K 36/481, A61P 25/00, A61P 13/00, A61P 9/00, A61K 36/286, A61K 36/537, A61K 36/65, A61K 36/69, A61K 36/736

(54) **NEUROPROTECTIVE COMPOSITION COMPRISING POLYGALAE, ASTRAGALI, CHUANXIONG AND ANGELICA SINENSIS**
NEUROPROTEKTIVE ZUSAMMENSETZUNG ENTHALTEND POLYGALAE, ASTRAGALI, CHUANXIONG UND ANGELICA SINENSIS
COMPOSITION NEUROPROTECTRICE COMPRENANT POLYGALAE, ASTRAGALI, CHUANXIONG ET ANGELICA SINENSIS

(30) Priority: 23.03.2012 US 201261614698 P; 15.03.2013 US 201361790851 P
(43) Date of publication of application: 25.02.2015
(73) Proprietor: Moleac Pte Ltd., Singapore 138667 (SG)
(72) Inventor: MOHA OU MAATI, Hamid, F-06000 Nice (FR); LAZDUNSKI, Michel, F-06000 Nice (FR); HEURTEAUX, Catherine, F-06480 La Colle sur Loup (FR); PICARD, David, Singapore 588292 (SG)
(74) Representative: Brevalex
(86) International application number: PCT/SG2013/000118
(87) International publication number: WO 2013/141818

(56) References cited:
- WO-A1-2007/106049
- WO-A1-2010/053456
- WO-A1-2010/110755
- US-A- 5 744 594
- C. HEURTEAUX ET AL: "Neuroprotective and neuroproliferative activities of NeuroAid (MLC601, MLC901), a Chinese medicine, in vitro and in vivo", NEUROPHARMACOLOGY., vol. 58, no. 7, 1 June 2010 (2010-06-01), pages 987-1001, XP055220620, GB ISSN: 0028-3908, DOI: 10.1016/j.neuropharm.2010.01.001
- SHERRY H.Y. YOUNG ET AL: "Safety Profile of MLC601 (Neuroaid ) in Acute Ischemic Stroke Patients: A Singaporean Substudy of the Chinese Medicine Neuroaid Efficacy on Stroke Recovery Study", CEREBROVASCULAR DISEASES, vol. 30, no. 1, 1 January 2010 (2010-01-01), pages 1-6, XP055220637, CH ISSN: 1015-9770, DOI: 10.1159/000313398
- QUINTARD H ET AL: "MLC901, a Traditional Chinese Medicine protects the brain against global ischemia", NEUROPHARMACOLOGY, PERGAMON PRESS, OXFORD, GB, vol. 61, no. 4, 5 May 2011 (2011-05-05), pages 622-631, XP028096961, ISSN: 0028-3908, DOI: 10.1016/J.NEUROPHARM.2011.05.003 [retrieved on 2011-05-14]
- HAI WANG ET AL: "Targeting Ischemic Stroke with a Novel Opener of ATP-Sensitive Potassium Channels in the Brain", MOLECULAR PHARMACOLOGY, AMERICAN SOCIETY FOR PHARMACOLOGY AND EXPERIMENTAL THERAPEUTICS, US, vol. 66, no. 5, 1 August 2004 (2004-08-01) , pages 1160-1168, XP008127087, ISSN: 0026-895X, DOI: 10.1124/MOL.104.003178
- MASHOUR N H ET AL: "Herbal medicine for the treatment of cardiovascular disease: Clinical considerations", ARCHIVES OF INTERNAL MEDICINE, AMERICAN MEDICAL ASSOCIATION, CHICAGO, IL, US, vol. 158, no. 20, 9 November 1998 (1998-11-09), pages 2225-2234, XP002103490, ISSN: 0003-9926, DOI: 10.1001/ARCHINTE.158.20.2225
- CHEN CHRISTOPHER L H ET AL: "The NeuroAiD II (MLC901) in vascular cognitive impairment study (NEURITES).", CEREBROVASCULAR DISEASES (BASEL, SWITZERLAND) 2013, vol. 35 Suppl 1, 2013, pages 23-29, XP002746853, ISSN: 1421-9786
- MOHA OU MAATI H ET AL: "Activation of ATP-sensitive potassium channels as an element of the neuroprotective effects of the Traditional Chinese Medicine MLC901 against oxygen glucose deprivation", NEUROPHARMACOLOGY, PERGAMON PRESS, OXFORD, GB, vol. 63, no. 4, 24 May 2012 (2012-05-24), pages 692-700, XP028400970, ISSN: 0028-3908, DOI: 10.1016/J.NEUROPHARM.2012.05.035 [retrieved on 2012-05-30]

## Description

### FIELD OF THE INVENTION

The present invention relates to the use of components of Traditional Chinese Medicine (TCM) for activation of ATP-sensitive potassium channels (K_{ATP} channels).

There is no admission that any of the various documents etc. cited in this text are prior art as to the present invention.

### INTRODUCTION

MLC 601 (NeuroAid) shows promising results in post-stroke recovery of patients (Chen et al., 2009). It combines 9 herbal and 5 animal components. A simplified formula of MLC601 called MLC901 (NurAid II, Neuroaid II or Neuroaid2) based on its 9 herbal components is also now available. MLC-601 treatment is currently used in several countries both in Asia and in the Middle East for stroke patients. A multicenter, randomized, double-blind placebo-controlled study to investigate CHInese Medicine MLC601 Efficacy on Stroke recovery (CHIMES) is ongoing in Asia (Venketasubramanian et al., 2009). The safety of MLC601 on hemostasis, hematology and biochemistry has been established both in normal subjects and stroke patients (Gan et al., 2008; Young et al., 2010). Its apparent efficiency in improving cerebral blood flow velocity (Shahripour et al., 2011) and stroke rehabilitation, even several months after stroke (Chen et al., 2009) are strong arguments to pursue fundamental studies to better understand in animal models as well as at the cellular and molecular levels how MLC901 works. We have previously demonstrated with rodents that both MLC601 and MLC901 improve survival, protect the brain from a focal ischemic injury and drastically decrease functional deficits induced by stroke (Heurteaux et al., 2010). Moreover in a model of global ischemia, mimicking a cardiac arrest, MLC901 has been shown to prevent both necrosis and apoptosis of hippocampal neurons and to improve both motor and cognitive recovery (Quintard et al., 2011). WO 2007/106049, WO 2010/110755, Sherry and Young, 2010 (Cerebrovascular Diseases, vol. 30, pages 1-6) and WO 2010/053456 disclose the use of Neuroaid and/or Neuroaid2 for the treatment of stroke and other disorders.

Among the many possible mechanisms of the beneficial effects of MLC601 or MLC901 against ischemia we have established that they stimulate BDNF expression, enhance neurogenesis, promote cell proliferation and stimulate neurite outgrowth. Moreover the Akt pathway, which is known to be involved in cell survival appears to be critical in the MLC901 protective effect, and the TCM prevents the exaggerated lipid oxydation produced by ischemia (Quintard et al., 2011).

The brain requires continuous oxygen and glucose supply to maintain function. Neurons are extremely vulnerable to hypoxic injury. In clinical conditions such as cerebral ischemia permanent loss of neuronal functions occurs within minutes of severe hypoxia. Ionic homeostasis (Na⁺, K⁺, Ca²⁺, Cl⁻) of brain tissue is greatly disturbed and neurons depolarize following oxygen deprivation leading to swelling, calcium overload, and subsequently to the death of neurons (Dirnagl et al., 1999; Lee et al., 2000, Obrenovitch, 2008). Because, under ischemic conditions, the intracellular ATP level falls substantially, because the ADP/ATP ratio is drastically increased, K_{ATP} channels are activated resulting in membrane hyperpolarization (Amoroso et al., 1990; Ashcroft and Ashcroft, 1990; Mourre et al., 1989). This hyperpolarization prevents for a short period a massive release of excitotoxic glutamate. A sustained activation of K_{ATP} channels has been proposed as a possible way to be neuroprotective against brain ischemia and indeed K_{ATP} openers have been shown to be neuroprotective (Blondeau et al., 2000; Heurteaux et al., 1993; Heurteaux et al., 1995; Lauritzen et al., 1997, Liss and Roeper, 2001).

The work described herein was designed to investigate the therapeutic effectiveness of MLC901 in a cellular model of oxygen glucose deprivation, which specifically mimics the rapid depletion of oxygen and glucose seen under ischemic conditions *in vivo.* The neuronal death induced oxygen glucose deprivation is inhibited by glibenclamide, an inhibitor of K_{ATP} channels. The activation of these channels enables cells to survive. Among interesting candidates for neuroprotection against ischemia, this work has demonstrated a very interesting activating effect on K_{ATP} channels.

ATP-sensitive potassium channels are a type of potassium channel that is inhibited by ATP and activated by ADP when the ADP level decreases. Because K_{ATP} channels have widespread and critical physiological functions, they have become drug targets. K_{ATP} channel openers (also known as KCOs or potassium channel openers (PCOs)) represent a structurally diverse group of compounds which act as activators (agonists) of K_{ATP} channel activity. Examples of PCOs include pinacidil, cromakalim and diazoxide.

### SUMMARY

The present invention relates to a composition or a kit consisting of four particular components selected in the Neuroaid2 components to activate K_{ATP} channels and for use in the treatment of various diseases and disorders as defined in the enclosed set of claims. The Neuroaid2 components are selected from the group consisting of: Salviae Miltiorrhizae, Prunus persica; Polygalae; acori tatarinowii; Astragali; Paeoniae Rubra; Chuanxiong; Carthamus tinctorius; and angelicae sinensis, and the selected Neuroaid2 components consist of Polygalae, Astragali, Chuanxiong, and angelicae sinensis.

Embodiments as defined in the present specification include the use of the Neuroaid2 components for activating ATP-sensitive potassium channels. Uses of the Neuroaid2 components include *in vivo, in vitro* and *ex vivo* uses. It is envisaged that by activating K_{ATP} channels that various diseases and conditions may be usefully treated. Examples of uses of the Neuroaid2 components and diseases / conditions which may be treated with the Neuroaid2 components : include obesity, incontinence (preferably urinary incontinence), hypertension, prevention of ischemic or reperfusion injury; treatment of hyperinsulemia or hyperinsulinism; treatment of hypoglycemia; prevention of the transition from prediabetes to diabetes; treatment of type II diabetes; correction of the defects in insulin secretion and insulin sensitivity contributing to prediabetes and type II diabetes; preservation of pancreatic function in type I diabetics; treatment of hyperlipidemia; prevention of weight gain in individuals who are predisposed to obesity; treatment of metabolic syndrome (or syndrome X); treatment of polycystic ovary syndrome; treat weight gain, dyslipidemia, or impairment of glucose tolerance in subjects treated with antipsychotics drugs; prevent weight gain, dyslipidemia, or impairment of glucose tolerance in subjects treated with antipsychotics drugs; treatment of any disease where hyperlipidemia, hyperinsulemia, hyperinsulinism, hyperlipidemia, hyperphagia or obesity are contributing factors to the severity or progression of the disease, including but not limited to, Prader Willi Syndrome, Froelich's syndrome, Cohen syndrome, Summit Syndrome, Alstrom, Syndrome, Borjesen Syndrome, Bardet-Biedl Syndrome, hyperlipoproteinemia type I, II, III, and IV; neurodegeneration; stroke; ischemia; epilepsy; pain; overactive bladder; irritable bowel syndrome; hair loss; baldness; alopecia; male erectile dysfunction; female sexual disorders; premature labor; benign prostate hyperplasia (BPH); dysmenorrheal; coronary artery disease; angina; airway hyperactivity; eating disorders; use as an anti-neoplastic agent (e.g. for treating brain cancers); skeletal muscle diseases such as myotonia congenita and hyperkalemic paralysis; use as a vasodilator; asthma; stopping the normal heartbeat in order to perform cardiac, aortic, neurovascular and cardiopulmonary organ transplant surgery and other related operations; impotence; treatment of arrhythmia; diabetes (e.g. diabetes mellitus type I or type II); insulin resistance; treating sensitive human skin; heart failure; peripheral vascular disorders; insulinoma; congenital hyperinsulinism; retinal ischemia; reducing the consumption of fat-containing foods; hypoglycaemia; depression or depression-related mood disorders; diseases / conditions benefiting from neuroprotection (see discussion below); and neuroconditioning. Other uses and diseases / conditions that may be usefully treated include those listed in WO2007/106049, WO2010/053456 and WO2010/110755.

### BRIEF DESCRIPTION OF DRAWINGS

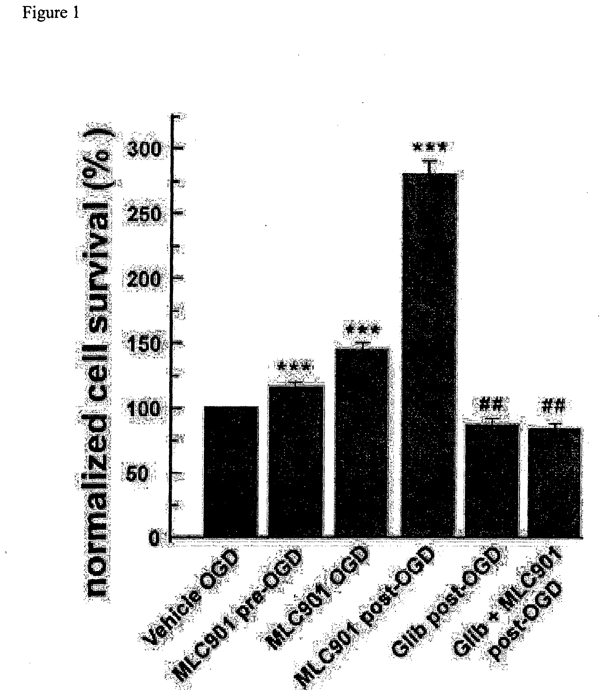

**Figure 1****. Neuroprotective effect of MLC901 on neuronal death induced by oxygen glucose deprivation (OGD) and its inhibition by glibenclamide**
   Cortical neurons in culture were treated with OGD during 2 hours. MLC901 (1 µg/mL) was applied two hours before OGD (pre-OGD), during OGD (OGD) or two hours after OGD (post-OGD). Glibenclamide was applied after OGD 30 min before MLC901. Histograms showing the normalized cell survival after MLC901 and glibenclamide treatments. Glibenclamide (10 µM) was applied alone or 30 min before MLC901. In both paradigms glibenclamide inhibited the protection induced by MLC901 against OGD damage (n = 12 dishes per experimental group,.****P<*0.001 versus vehicle group and ^{##}*P*<0.001 versus MLC901 group).
**Figure 2****. Effect of MLC901 on endogenous K_{ATP} currents in INS-R9 cells.** Current traces recorded in control conditions and in the presence of MLC901(µg/ml) alone, MLC901 (1µg/ml) + Pinacidil (10 µM) or MLC901(1µg/ml) + Pinacidil (10 µM) + Glibenclamide (10 µM).
**Figure 3****. Effect of MLC901 on K_{ATP} channels (Kir 6.1/SUR2) expressed in Xenopus oocytes.**
   **(A)** Typical current traces recorded in control conditions (90 K, n = 6), in the presence of Azide (3 mM) alone or Azide + Pinacidil (10 µM) or Azide + Pinacidil + Glibenclamide (10 µM) (n = 6 per condition). **(B)** Typical current traces recorded in control conditions (90 K, n = 6), and in the presence of Azide (3mM) alone, Azide + MLC901 (1 µg/mL) or Azide + MLC901 (1 µg/mL) + Glibenclamide (10 µM) (n = 6 per condition). **(C)** Whole-cell current-voltage relationships obtained with voltage ramps ranging from -160 to + 80 mV in control conditions (90K), and in the presence of Azide (3mM) alone, Azide + MLC901 (1 µg/mL) or Azide + MLC901 (1 µg/mL) + Glibenclamide (10 µM) (n = 6 per condition). **(D)** Corresponding histograms showing the current amplitude at -120 mV for all tested conditions (n = 6; ***P*<0.01 and ****P*<0.001).

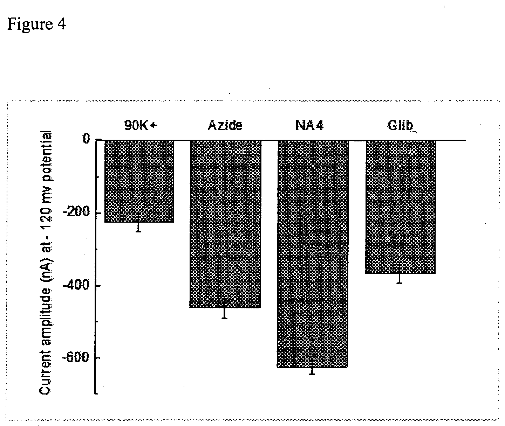

**Figure 4****: Effects of NA4 (1µg / ml) on KATP currents in Xenopus oocytes (n=4)** Currents were recorded in the potassium symmetrical control condition. K_{ATP} channel activity was evaluated by intracellular ATP depletion by 3 mM of sodium azide.
   Perfusion protocol: current was first recorded in control condition (symmetrical potassium solution) (90K+). Then, channel activity was enhanced by 6 minutes of 3 mM sodium azide perfusion which was maintained during the entire experiment. MLC901 or other batch products were perfused 6 minutes after sodium azide perfusion. In all experiments, inhibition of recorded currents by Glibenclamide (glib) (10 µM) was evaluated.

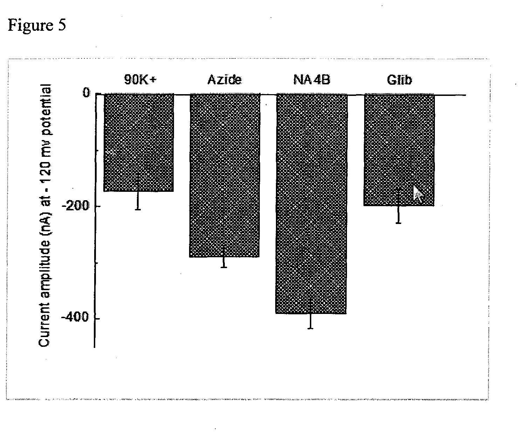

**Figure 5****: Effects of NA4 (1µg / ml) on KATP currents in oocytes (n=4)**
   Currents were recorded in the potassium symmetrical control condition. K_{ATP} channel activity was evaluated by intracellular ATP depletion by 3 mM of sodium azide.
   Perfusion protocol: current was first recorded in control condition (symmetrical potassium solution). Then, channel activity was enhanced by 6 minutes of 3 mM sodium azide perfusion which was maintained during the entire experiment. MLC901 or other batch products were perfused 6 minutes after sodium azide perfusion. In all experiments, inhibition of recorded currents by Glibenclamide (glib) (10 µM) was evaluated.
**Figure 6****:**
   The 2 samples, NeuroAid2 (our control with the 9 plants) and "NA3" (chuanxiong, sinensis, astragalus (radix astragali)) are activating the channel.

### GLOSSARY OF TERMS

This section is intended to provide guidance on the interpretation or scope of the words and phrases set forth below (and where appropriate grammatical variants thereof). Further guidance on the interpretation or scope of certain words and phrases as used herein (and where appropriate grammatical variants thereof) may additionally be found in other sections of this specification.

The words "a", "an" and "the" are employed to describe elements and components of the invention. This is done merely for convenience and to give a general sense of the invention. This description should be read to include one or at least one and the singular also includes the plural, unless the context clearly indicates otherwise. Thus, for example, the term "an agent" includes a reference to a single agent as well as a plurality of agents (including mixtures of agents). It should also be noted that the term "or" is generally employed in its sense including "and/or" unless the content clearly dictates otherwise.

As used herein, the term "about" as used in relation to a numerical value means, for example, ±25% of the numerical value, preferably ±15%, more preferably ±10%, more preferably still ±5%, and most preferably ±2% or ±1%. Where necessary, the word "about" may be omitted from the definition of the invention.

By "a cerebral stroke patient" we include a patient who has suffered an ischemic or haemorrhagic cerebral stroke. A cerebral stroke is a sudden and permanent death of brain cells that occurs when the flow of blood is blocked and oxygen cannot be delivered to the brain, ischaemic stroke most commonly occurs when the flow of blood is prevented by clotting (known as 'thrombosis' of the artery) or by a detached clot that lodges in an artery (referred to as an 'embolic stroke'). Haemorrhagic stroke results from rupture of an artery wall, and from blood leaking into the surrounding brain. Haemorrhagic stroke, like ischemic stroke, causes the death of tissue by depriving the brain of blood and oxygen, and results in a number of neurological disabilities (motor, speech) as well as functional disabilities.

As used herein, the term "comprising" means "including". Thus, for example, a composition "comprising" Neuroaid2 components may consist exclusively of the Neuroaid2 components or it may include one or more additional components (e.g. a further active agent, a pharmaceutically acceptable carrier or excipient etc). Thus, it is to be understood that the term "comprising" includes within its scope embodiments where one or more additional, unrecited elements are present, as well as the more restrictive terms "consisting essentially of' and "consisting of' (i.e. where only the recited elements are present). The term "including" is to be likewise interpreted so as to encompass "including, but not limited to ... ", and the more restrictive terms "consisting essentially of" and "consisting of."

The term "extraction" as used herein includes a reference to a method of separation in which plant material (e.g. chopped parts of a plant, whether fresh or dried) is contacted with a liquid solvent to transfer one or more components of the plant material into the solvent.

The term *"in vivo"* as used herein includes a reference to using a whole, living organism. This contrasts with the term *"in vitro"* where a whole, living organism is not used. The term *"in vitro"* is to be understood as including, inter alia, *"ex vivo"* uses whereby cells, tissue etc. which does not form part of a whole, living organism may be employed (e.g. cells or tissues from cell or tissue cultures, biopsies, dead organisms etc.). Further non-limiting examples of *"in vitro"* relate to the use of cellular extracts or lysates.

The terms "patient" and "subject" are used interchangeably herein and the terms include a reference to any human or non-human animal (preferably a mammal) that it is desired to treat using the present invention. However, it will be understood that "patient" or "subject" does not imply that symptoms are present. The term "mammal" as used herein refers to any member of the class Mammalia, including, without limitation, humans and non-human primates such as chimpanzees and other apes and monkey species; farm animals such as cattle, sheep, pigs, goats and horses; domestic / companion animals such as dogs and cats; laboratory animals (e.g. rabbits and rodents such as mice, rats, and guinea pigs, and the like). Preferably, the mammal is human.

The term "stroke" refers to the sudden death of tissue cells due to a lack of oxygen when the blood flow is impaired by blockage or rupture of an artery. Stroke is a vascular accident that can occur in the brain or in the cardiac system. The latter condition is medically known as "myocardial infarction" and more commonly known as a "heart attack".

The term "treatment" as used herein is intended to be construed broadly and includes a reference to any and all uses which remedy a disease state or symptoms (e.g. reduce the severity of the disease or symptoms, reduce the frequency of symptoms etc), prevent the establishment of disease, or otherwise prevent, hinder, retard, or reverse the progression of disease or other undesirable symptoms in any way whatsoever, even if the treatment is ultimately unsuccessful. Treatment may be in respect of a patient which already has the disorder, or in respect of a patient which is prone to have the disorder or in whom the disorder is to be prevented. Thus, the term "treatment" (and for the avoidance of doubt, grammatical variants thereof such as "treating" etc.) may refer to therapeutic treatment or to prophylactic or preventative treatment.

The terms "disease", "disorder" and "condition" may be used herein interchangeably, unless the context clearly dictates otherwise.

Unless otherwise indicated, all technical and scientific terms used herein have the same meaning as commonly understood to one of ordinary skill in the art to which the invention belongs.

### DETAILED DESCRIPTION

One aspect as defined in the present specification provides four Neuroaid2 components for use in a method of treating a disease / disorder in a patient wherein the disease / disorder can be treated by activating K_{ATP} channels and wherein the four Neuroaid2 components consist of Polygalae, Chuanxiong, preferably the rhizome thereof (i.e. rhizoma chuanxiong); angelicae sinensis (Chinese Angelica or DanGui), preferably the root (i.e. radix angelicae sinensis); and Astragali (Membranous Milkvetch or Huang Qi), preferably the root (i.e. radix astragali).

Another aspect as defined in the present specification provides a composition or kit comprising four Neuroaid2 components consisting of Polygalae, Chuanxiong, preferably the rhizome thereof (i.e. rhizoma chuanxiong); angelicae sinensis (Chinese Angelica or DanGui), preferably the root (i.e. radix angelicae sinensis); and Astragali (Membranous Milkvetch or Huang Qi), preferably the root (i.e. radix astragali).

The above aspects as defined in the present specification are described in more detail below with the following sections being intended to provide guidance on the implementation and scope of the various aspects as defined in the present specification and scope of the invention as defined in the enclosed set of claims.

### Activation of K_{ATP} Channels

The various aspects as defined in the present specification relate to the activation of K_{ATP} channels by Neuroaid2 components. By using the Neuroaid2 components, K_{ATP} channels may be activated in one or more cell types. For example, K_{ATP} channels may be activated in one or more of the following cell types: neuronal cells, smooth muscle cells (e.g. vascular smooth muscle cells, smooth muscle cells of the bladder), cardiac myocytes, pancreatic beta cells, adipose tissue cells and skeletal muscle cells. In at least some embodiments of the various aspects as defined in the present specification, the Neuroaid2 components are for activating K_{ATP} channels in cells which are not neuronal cells. K_{ATP} channels are found in various cellular locations including the plasma membrane, mitochondria ("mitoK_{ATP}") and nuclear ("nucK_{ATP}"). It is envisaged that the Neuroaid2 components may be used for the activation of K_{ATP} channels in one or more of these locations. However, in at least some embodiments there is provided the use of the Neuroaid2 components for activating plasma membrane K_{ATP} channels. In at least some embodiments of the invention there is provided the use of the Neuroaid2 components for activating sarcolemmal K_{ATP} channels.

The Neuroaid2 components are for use in various methods of treatment by the activation of KATP channels.

### The Neuroaid2 Components

The Neuroaid2 components are:
1. Salviae Miltiorrhizae (Red Sage or Dan Shen), for example the root (radix) and/or rhizome (rhizoma) thereof (in preferred embodiments, the radix, or radix et rhizome);
2. Prunus persica (Peach or Tao ren), preferably the seed thereof;
3. Polygalae (thinleaf milkwort, Polygala tenuifolia Willd., Polygala sibirica L. or Yuanzhi), preferably the root thereof (i.e. radix polygalae);
4. acori tatarinowii (grassleaf sweetflag or Shichangpu), preferably the rhizome thereof (i.e. rhizoma acori tatarinowii);
5. Astragali (Membranous Milkvetch or Huang Qi), preferably the root (i.e. radix astragali);
6. Paeoniae Rubra (Red Peony, Paeonia lactiflora Pall, Paeonia veitchii Lynch or Chi Shao), preferably the root (i.e. radix paeoniae rubra);
7. Chuanxiong; preferably the rhizome thereof (i.e. rhizoma chuanxiong);
8. Carthamus tinctorius (Safflower or HongHua), preferably the flower thereof; and
9. Angelicae sinensis (Chinese Angelica or DanGui), preferably the root (i.e. radix angelicae sinensis).

The four Neuroaid2 components selected from the above 9 Neuroaid2 components and which consist of Polygalae, Chuanxiong, angelica sinensis and Astragali are herein referred to as "the selected Neuroaid2 components".

Combinations of Neuroaid2 components may suitably be additive or synergistic. An "additive" effect refers to a beneficial pharmaceutical effect produced by the combination which is larger than the effect of any of the components of the combination when presented individually. A "synergistic" effect refers to a beneficial pharmaceutical effect produced by the combination which is larger than the sum of the effects of the components of the combination when presented individually.

The various aspects as defined in the present specification employ four of the above-mentioned Neuroaid2 components consisting of Polygalae, Chuanxiong, preferably the rhizome thereof (i.e. rhizoma chuanxiong); Angelicae sinensis (Chinese Angelica or DanGui), preferably the root (i.e. radix angelicae sinensis); and Astragali (Membranous Milkvetch or Huang Qi), preferably the root (i.e. radix astragali).

In the present invention only 4 Neuroaid2 components are employed and the latter are Polygalae, Chuanxgiong, Angelica sinensis, and Astragali.

In the invention the selected Neuroaid2 components consist of:
i. Astragali (Membranous Milkvetch or Huang Qi), preferably the root (i.e. radix astragali).
ii. Chuanxiong; preferably the rhizome thereof (i.e. rhizoma chuanxiong).
iii. Angelicae sinensis (Chinese Angelica or DanGui), preferably the root (i.e. radix angelicae sinensis); and
iv. Polygalae (thinleaf milkwort, Polygala tenuifolia Willd., Polygala sibirica L. or Yuanzhi), preferably the root thereof (i.e. radix polygalae).

### Neuroaid and NeuroaidII

The Neuroaid2 components are the nine herbal components of NeuroAid II (also known as NurAid II, Neuroaid2, and MLC901). The nine herbal components of NeuroAid II are:

| |
|---|
| *Radix astragali* |
| *Radix salviae miltiorrhizae* |
| *Radix paeoniae rubra* |
| *Rhizoma chuanxiong* |
| *Radix angelicae sinensis* |
| *Carthamus tinctorius* |
| *Semen persica* |
| *Radix polygalae* |
| *Rhizoma acori tatarinowii* |

Neuroaid II comprises the following along with dextrin or maltodextrin as excipient:

| **MLC901 components** | ***Qty (mg) per capsule** |
|---|---|
| *Radix astragali* | 800 |
| *Radix salviae miltiorrhizae* | 160 |
| *Radix paeoniae rubra* | 160 |
| *Rhizoma chuanxiong* | 160 |
| *Radix angelicae sinensis* | 160 |
| *Carthamus tinctorius* | 160 |
| *Semen persica* | 160 |
| *Radix polygalae* | 160 |
| *Rhizoma acori tatarinowii* | 160 |

NeuroAid™ (MLC601) from Moleac Pte Ltd is a TCM product in capsule form comprising 9 herbal components and 5 animal components. NeuroAid™ comprises Radix Astragali (root of Membranous Milkvetch or Huang Qi), Radix et Rhizoma Salviae Miltiorrhizae (Red Sage root or Dan Shen), Radix Paeoniae Rubra (Red Peony root, Paeonia lactiflora Pall, Paeonia veitchii Lynch or Chi Shao), rhizome of Ligusticum Chuanxiong (Chuan Xiong), radix angelicae sinensis (root of Chinese Angelica or DanGui), Flower of Carthamus Tinctorius (Safflower or HongHua), Prunus Persica (Peach seed or Taoren), Radix Polygalae (root of thinleaf milkwort, Polygala tenuifolia Willd., Polygala sibirica L. or Yuanzhi), Rhizoma acori Tatarinowii (rhizome of grassleaf sweetflag or Shichangpu), Buthus martensii (dried body of scorpio or Quanxie), dried body of leeches (Hirudo, Whitmania pigra Whitman, Hirudo nipponica Whitman, Whitmania acranulata Whitman or Shuizhi),

Eupolyphaga Seu Seteleophaga (dried body of ground beetle, Eupolyphaga sinensis Walker, Steleophaga plancyi or Tubiechong), Calculus Bovis Artifactus (Natural or Artificial cow-bezoar or Rengong Niuhuang), and Cornu Saigae Tataricae (Antelope Horn or Lingyangjiao).

The composition of MLC601 is shown in the table below. MLC601 comprises the following along with dextrin as an excipient.

| Latin Name | Quantity |
|---|---|
| Radix Astragali | 0.570 g |
| Radix salviae miltiorrhizae | 0.114 g |
| Radix paeoniae rubra | 0.114 g |
| Rhizoma chuanxiong | 0.114 g |
| Radix angelicae sinensis | 0.114 g |
| Carthamus tinctorius | 0.114 g |
| Hirudo | 0.0665 g |
| Eupolyphaga seu steleophaga | 0.0665 g |
| Prunus persica | 0.114 g |
| Calculus bovis artifactus | 0.0285 g |
| Cornu saigae tataricae | 0.0285 g |
| Buthus martensii | 0.095 g |
| Radix polygalae | 0.114 g |
| Rhizoma acori tatarinowii | 0.114 g |

NeuroAid™, which may be registered under different names in different countries (e.g. Nuraid, Nu-raid, in South Africa it is marketed as Strocaid ™, or Dangi Piantan Jiaonang ™) is manufactured by and available commercially in the People's Republic of China from Tianjin Shitian Pharmaceutical Group Co., Ltd (located in the Jianxin Industrial area, Wangwenzhuang town, Xiqing district, Tianjin City, China; Postal Code 300381). It is also available from Moleac Pte Ltd (formerly Molecular Acupuncture Pte Ltd), the main licensee outside of the People's Republic of China (11 Biopolis Way, Helios #09- 08 Singapore 138667).

For the avoidance of doubt, NeuroAid™ and NeuroAid II not only includes NeuroAid™ and NeuroAidII respectively in the forms in which it is currently marketed but also includes future formulations of NeuroAid™ and NeuroAid II respectively which may, for example, be marketed by Tianjin Shitian Pharmaceutical Group Co., Ltd or Moleac Pte Ltd. Such future formulations may, for example, vary in dosage amounts or the concentration of its active ingredients etc.

NeuroAid™ is also known as MLC 601 and the terms "NeuroAid™" and "MLC 601" can be used interchangeably. Similarly NeuroAid II is also known as MLC 901, Neuroaid2, NurAid II, NeuroaidII, Regenaid and Nu-raidII and these terms can be used interchangeably.

### Uses of the Neuroaid2 Components

Various uses have been described in the art for Neuroaid and related compositions. See for example WO2007/106049, WO2010/053456 and WO2010/110755 (all to Moleac Pte. Ltd.), in particular in relation to the uses described therein for Neuroaid and related compositions.

WO2007/106049 and WO2010/053456 describe the use of Neuroaid and related compositions for treating stroke (e.g. cerebral stroke and cardiovascular disease (heart stroke mainly due to coronary artery stroke)), neurological disorders, treating cerebral infarct patients, brain trauma, nervous system trauma, conditions related to neuroplasticity, head trauma, cardiac arrest, subarachnoid hemorrhage, apoplexy and its use as a dietary supplement to provide nutrition to healthy individuals as well as patients afflicted with stroke or neurological disorders.

Neurological disorders are disorders that affect the central nervous system, the peripheral nervous system and the autonomic nervous system such as neurodegenerative diseases (for example, Alzheimer's disease and Parkinson's disease), epilepsy, seizure, demyelinating diseases (for example, multiple sclerosis), cerebral palsy, traumatic injuries to or tumours in the brain, spinal cord and peripheral nerves.

Neuroplasticity (also referred to as brain plasticity or cortical plasticity) refers to changes that occur in the organization of the brain and its circuits of neurons, in particular changes that occur to the location of specific information processing functions. This process supports the learning of new functions as the result of experience during development as mature animals and the creation of new information with healthy neurons by-passing damaged neurons affected by trauma or a medical condition.

WO2010/110755 includes experimental data relating to MLC601 and MLC901. Positive effects on cell viability, LDH release, ischemic brain injury, neuroproliferation and neurite outgrowth are demonstrated therein for MLC601 and MLC901.

WO2010/110755 discloses the use of Neuroaid and related compositions for promoting neuronal outgrowth and proliferation of neurons or stem cells, such as in injured or diseased tissues. The term "neuronal outgrowth" relates to the general directional outward growth of axons and dendrites. Neuronal outgrowth is important in synapse formation or development. WO2010/110755 discloses the treatment of patients having a condition selected from the group consisting of: psychiatric indications such as anxiety disorders, schizophrenia, depression, and post-natal depression, natural aging, traumatic brain cell death and other neurologic manifestations such as amnesia, back pain, vertigo, unconsciousness, phantom limb, olfaction disorders, neck pain, headache, migranes, spasm and speech disorders. Other uses which are disclosed include the treatment of: neurodegeneration; nervous system injuries or diseases (e.g. amyotrophic lateral sclerosis (ALS) and senile dementia); neurological diseases, degenerative diseases; stroke; rheumatoid arthritis, muscle degenerative disorders, kidney diseases, and liver diseases.

WO2010/110755 also discloses the use of Neuroaid and related compositions for: the general well being of neurons; promoting cell survival, proliferation or differentiation of cells (e.g. to thereby facilitate the *in vitro* culture of various cells that may be used for tissue engineering or ex vivo therapeutic uses); promoting stem cell differentiation and recruitment into diseased or injured tissue; treating a patient having diseased or injured tissue of the central or peripheral nervous system; promoting cell growth; promoting controlled growth of chondrocytes, skeletal muscle cells, myocardiums, smooth muscle cells, hepatocytes, kidney cells or epithelial skin cells; delaying aging processes by improving epithelial or epidermal cell proliferations or functions; promoting cell survival and growth of neurons, stem cells, chondrocytes, skeletal muscle cells, myocardiums, smooth muscle cells, hepatocytes, kidney cells, islets of langerhans and epithelial skin cells.

In at least some embodiments of the invention, the composition or the kit consisting of four particular components selected in the Neuroaid2 components for activating KATP channels is for use in a method for providing neuroprotection.

The term "neuroprotection" as used herein includes a reference to the preservation of neuronal tissue at risk of dying, such as during stroke or in the aftermath of a stroke. The term "neuroprotection" (and for the avoidance of doubt, grammatical variants thereof) may accordingly refer to the stimulation or promotion of cell survival, or prevention of cell death, where the cell is at risk of cell death, such as where the cell has been traumatised and would under normal circumstances (i.e. without intervention / treatment), with a high probability die. Accordingly, it will be understood that the terms "neuroprotection", stimulation or promotion of cell survival, and the prevention of cell death may be used synonymously, unless the context indicates otherwise.

Neuroprotection can be used to protect cells from stress (e.g. lack of oxygen, lack of glucose, glutamate stress, free radicals) within the nervous system, such as within the brain. Also by promoting survival it is possible to prevent or slow down diseases or prevent or slow down further degeneration of the nervous system in individuals suffering from a degenerative disorder.

Various factors may put cells at risk of dying with examples including: traumas, injuries, acute diseases and/or disorders, chronic diseases and/or disorders such as neurodegenerative diseases. The neurodegeneration may be caused by diseases selected from the group of Alzheimer's disease, amyotrophic lateral sclerosis (ALS), brain abscess, brain ischemia, brain atrophy associated with diabetes, cerebral autosomal dominant arteriopathy with subcortical infarcts and leukoencephalopathy (CADASIL), cerebrovascular disorders, corticobasal ganglionic degeneration (CBGD), chronic ischemia, Creutzfeldt-Jakob Syndrome, Dandy-Walker Syndrome, Duchenne Muscular Dystrophy, senile dementia, dementia associated with Acquired Immunodeficiency Syndrome (AIDS), encephalomyelitis, essential tremor, friedreich ataxia, gerstmann straussler-scheinker disease, Huntington disease, hydrocephalus, hypoxia, fatal familial insomnia, transient ischemic attack, kuru, Landau-Kleffner Syndrome, Lewy body disease, Machado-Joseph disease, bacterial and viral meningitis, migraine disorders, myelitis, olivopotocerebellar atrophies, pantothenate kinase-associated neurodegeneration, Parkinson's disease, poliomyelitis, postpoliomyelitis syndrome, prion diseases, pseudotumor cerebri, shy-drager syndrome, Steinert disease, infantile spasms, progressive supranuclear palsy, syringomyelia, thalamic diseases, Tic disorders, Tourette syndrome, Uveomeningoencephalitic syndrome, global and focal ischemia and other cardiovascular diseases, in predisposed subjects. External factors may also put cells at risk of dying, such as infections, toxic exposure (e.g. radiation, chemical, or drug-related), medical or surgical treatment (e.g. opening of the cranial cavity). Diagnostic methods can also put cells at risk of dying since diagnostic methods may cause formation of free radicals or otherwise have cytotoxic effects, such as X-rays and chemotherapy. Accordingly, in at least some embodiments the cells are at risk of dying due to an infection, toxic exposure (e.g. radiation, chemical, or drug-related), or from medical, surgical or diagnostic treatment.

The four selected Neuroaid2 components may be used for promoting the survival of cells of the nervous system which are at risk of dying due to a disease, disorder, injury or condition of the central (brain or spinal cord) and/or peripheral nervous system. Examples include cancer of the neural system, postoperative nerve damage, traumatic nerve damage, e.g. resulting from spinal cord injury, impaired myelination of nerve fibers, postischaemic damage, e.g. resulting from a stroke, multiinfarct dementia, multiple sclerosis, nerve degeneration associated with diabetes mellitus, neuro-muscular degeneration, schizophrenia, depression (including e.g. post-natal depression, bipolar disorder, major depression etc.), psychiatric disorders, natural aging, Alzheimer's disease, Parkinson's disease, dementia or Huntington's disease, and migraines.

The four selected Neuroaid2 components may be used to promote the survival of cells of the nervous system which are at risk of dying due to brain injury, stroke, ischemia, migraine, spinal cord injury, a neurodegenerative disease/disorder/condition, dementia (e.g., Alzheimer's disease, Parkinson's disease, Huntinton's chorea and spinocerebreallar degeneration) or a psychiatric disorder such as depression.

In a preferred embodiment of the invention, the composition or the kit consisting of four particular components selected from the Neuroaid2 components is for use in the treatment of stroke. The patient may have just had a stroke (e.g. within the past 96, 84, 72, 60, 48, 36, or 12 hours) or may be at risk of a stroke, such as a patient who is at risk of a first stroke or is at risk of a recurrence of stroke. Where the Neuroaid2 components are for treating stroke, the components may optionally be for administration to the patient within about 96, 84, 72, 60, 48, 36, or 12 hours of a stroke, such as within about 1 to 12 hours after a stroke; about 2 to about 10 hours after a stroke; or more preferably within about 3 to about 9 hours after a stroke. Optionally the Neuroaid2 components are for administration to the patient within about 10, 9, 8, 7, 6, 5, 4, 3, 2 or 1 hours after a stroke. Where the Neuroaid2 components may be used to treat a patient who is at risk of a recurrence of stroke, the Neuroaid2 components may be for administration at least about 1, 2, 3, 4, 5, 6 or 7 days after the previous stroke, or at least about 1, 2, 3, 4, 5, or 6 weeks after the previous stroke, or at least about 1, 2, 3, 4, 5, 6, 7, 8, 9, 10, 11 or 12 months after the previous stroke.

It is envisaged that the Neuroaid2 components will find utility in respect of a use described in the art for ATP-sensitive potassium channel activators (also known as PCOs and KCOs), such as for one or more of pinacidil, cromakalim and diazoxide. Examples of such uses are provided below and in a preferred embodiment of the various aspects disclosed in the present specification the Neuroaid2 components are employed for a use which is listed below.

Various uses of K_{ATP} channel openers are described in WO2006026469 and these include: prevention of ischemic or reperfusion injury; treatment of hyperinsulemia or hyperinsulinism; treatment of hypoglycemia; prevention of the transition from prediabetes to diabetes; treatment of type II diabetes; correction of the defects in insulin secretion and insulin sensitivity contributing to prediabetes and type II diabetes; preservation of pancreatic function in type I diabetics; treatment of hyperlipidemia; prevention of weight gain in individuals who are predisposed to obesity; treatment of obesity; treatment of metabolic syndrome (or syndrome X); treatment of polycystic ovary syndrome; treatment of weight gain, dyslipidemia, or impairment of glucose tolerance in subjects treated with antipsychotics drugs; prevention of weight gain, dyslipidemia, or impairment of glucose tolerance in subjects treated with antipsychotics drugs; and treatment of any disease where hyperlipidemia, hyperinsulemia, hyperinsulinism, hyperlipidemia, hyperphagia or obesity are contributing factors to the severity or progression of the disease, including but not limited to, Prader Willi Syndrome, Froelich's syndrome, Cohen syndrome, Summit Syndrome, Alstrom, Syndrome, Borjesen Syndrome, Bardet-Biedl Syndrome, or hyperlipoproteinemia type I, II, III, and IV.

US 8,053,441 also describes a number of diseases or conditions which may be treated with potassium channel openers including: hypertension, neurodegeneration, stroke, ischemia, epilepsy, pain, overactive bladder, urinary incontinence, irritable bowel syndrome, hair loss, baldness, alopecia, male erectile dysfunction, female sexual disorders, premature labor, benign prostate hyperplasia (BPH), dysmenorrhea, coronary artery disease, angina, and airway hyperactivity eating disorders. Other uses for K_{ATP} channel openers include: use as an anti-neoplastic agent, particularly for the use of brain cancers (US 7,705,010, and the treatment of skeletal muscle diseases such as myotonia congenita and hyperkalemic paralysis (US 5,744,594).

Preferably, the Neuroaid2 components are employed for a use described in the art for pinacidil. Uses of pinacidil (N-cyano-N'-pyridin-4-yl-N"-(1,2,2-trimethylpropyl)guanidine) include: use as a vasodilator, to treat hypertension, asthma, urinary incontinence, stopping the normal heartbeat in order to perform cardiac, aortic, neurovascular and cardiopulmonary organ transplant surgery and other related operations (in respect of the aforementioned uses of pinacidil see e.g. US5,428,039), treating sensitive human skin (see e.g. US 6,572,848) and impotence (see e.g. US 7,959,550).

Preferably, the Neuroaid2 components are employed for a use described in the art for cromakalim. Uses of cromakalim ((3R,4S)-3-hydroxy-2,2-dimethyl-4-(2-oxopyrrolidin-1-yl)chroman-6-carbonitrile) include: use as a vasodilator, to treat hypertension (see e.g. US 7,964,623), asthma (see e.g. US 7,964,623), treatment of arrhythmia (see e.g. US 7,964,623), diabetes (e.g. diabetes mellitus type I or type II), obesity (see e.g. US 7,964,623), metabolic syndrome, syndrome X, insulin resistance, stopping the normal heartbeat in order to perform cardiac, aortic, neurovascular and cardiopulmonary organ transplant surgery and other related operations (in respect of the aforementioned uses of cromakalim see e.g. US 5,428,039) treating sensitive human skin (see e.g. US 6,572,848), heart failure and impotence (US 7,959,550).

Preferably, the Neuroaid2 components are employed for a use described in the art for diazoxide. Diazoxide (brand name Proglycem, 7-chloro-3-methyl-4H-1,2,4-benzothiadiazine 1,1-dioxide). Uses include: use as a vasodilator, to treat hypertension (see e.g. US. 2,986,573), peripheral vascular disorders (see e.g. U.K. Patent GB982072) insulinoma, congenital hyperinsulinism, metabolic syndrome (in respect of these uses see e.g. US 5,284,845 or US 6,197,765), retinal ischemia (see e.g. US 8,063,054), diabetes (see e.g. US 7,799,777), obesity (see e.g. US 7,799,777 and U.S. Patent Publication No. 2004/0204472, treatment of syndrome X (see e.g. U.S. 6,197,765), and reducing the consumption of fat-containing foods (U.S. Patent publication no. 2003/0035106), and hypoglycaemia.

US 8,101,600 discloses that pinacidil, cromakalim and diazoxide may be used to treat depression or depression-related mood disorders (the term "mood disorders" being used to encompass those conditions defined as mood disorders in the Diagnostic and Statistical Manual of Mental Disorders, Fourth Edition, 1994 ("DSM-IV")). US 7,018,979 discloses that pinacidil, cromakalim and diazoxide may be useful in selectively delivering a medicament to a malignant tumor in the brain or to a tumor elsewhere in the body of a mammalian subject.

It is envisaged that the Neuroaid2 components may be employed for a use as described above in relation to KCOs, pinacidil, cromakalim or diazoxide. With regard to the above-mentioned uses of K_{ATP} channel activators and pinacidil, diazoxide and cromakalim various publications in the art may be consulted including, for example US5,744,594; WO2006026469; US 8,101,600; US 8,058,264; US 8,053,441; US 7,799,777; US 7,115,620; US 6,417,207 as well as the publications listed above.

In at least some embodiments disclosed in the present specification, the Neuroaid2 components are used for treating a condition selected from the group consisting of: incontinence (preferably urinary incontinence), obesity and hypertension.

In at least some embodiments disclosed in the present specification, the Neuroaid2 components are used for treating incontinence, preferably urinary incontinence.

The use of KCOs in treating urinary incontinence is described in, inter alia, US 8,053,441, US 7,115,620 and US 6,417,207; and the use of pinacidil in the treatment of incontinence is described, inter alia, EP260,790.

In at least some embodiments disclosed in the present specification, the Neuroaid2 components are used for treating obesity. The use of KCOs and diazoxide in treating obesity is described in, inter alia, WO2006026469 and US7,799,777.

In at least some embodiments disclosed in the present specification, the Neuroaid2 components are used for treating hypertension. KCOs have been characterized as having potent antihypertensive activity *in vivo* and vasorelaxant activity *in vitro* (Quast, U., et. al., Cellular Pharmacology of Potassium Channel Openers in Vascular Smooth Muscle, Cardiovasc. Res., Vol. 28, pp. 805-810 (1994)). US 8,053,441 describes the use of KCOs in treating hypertension; US7,115,620 describes the use of pinacidil and cromakalim in treating hypertension; WO2006026469, US 8,063,054, U.S.2,986,573 and US 7,799,777 describe the use of diazoxide in treating hypertension; and US 7,964,623 describes the use of cromakalim in treating hypertension.

In at least some embodiments of the invention, the composition or the kit consisting of four particular components selected from the Neuroaid2 components is used for providing neuroconditioning.

The term "neuroconditioning " as used herein includes a reference to pharmacologically induced molecular events preventing or reducing possible future brain damage. Neuroconditioning results in providing tolerance to the brain against an ischaemic, epileptic or other injurious event. The effect is similar to preconditioning (a clinical and experimental approach demonstrated to be effective), but does not require exposure to stressful stimuli. Neuroconditioning induces prophylactically a tolerance in a patient, such as those at risk of an insult. Examples of such insults include insults resulting from a condition selected from the group consisting of: ischemic, transient or permanent, focal or generalized; seizure, focal or generalized; inflammatory; toxic (e.g. radiation, chemical, or drug-related); immunologic; infectious; metabolic, nutritional; traumatic; compressive; neoplastic; degenerative; genetic, congenital; and procedural (including for example those requiring general anesthesia, clamping of major vessels, or opening of the cranial cavity). Thus, in at least some embodiments of the invention the Neuroaid2 components are for administration to a patient which is at risk of an insult such as, for example, an insult resulting from a condition selected from the group consisting of: ischemic, transient or permanent, focal or generalized; seizure, focal or generalized; inflammatory; toxic (e.g. radiation, chemical, or drug-related); immunologic; infectious; metabolic, nutritional; traumatic; compressive; neoplastic; stroke, degenerative; genetic, congenital; and procedural (including for example those requiring general anesthesia, clamping of major vessels, or opening of the cranial cavity).

### Preparation of the Neuroaid2 Components

As indicated above, Neuroaid2 comprises nine different herbal components. The plants used to obtain the herbal components may be used in their entirety (i.e. the whole plant is used) or one or more parts of the plants may be used. Parts of the plants that may be used include: the leaves, flowers, stems, roots, seeds, spores, stalks, rhizomes, fruit, fruiting bodies, and mixtures of said plant parts. Unless the context indicates otherwise, the term "plant" is intended to encompass whole plants as well as one or more parts thereof including the leaves, flowers, stems, roots, seeds, spores, stalks, rhizomes, fruit, and fruiting bodies.

In at least some embodiments of the invention, where the rhizome of a particular plant is indicated as being the preferred part of the plant (e.g. rhizoma chuanxiong) the root (radix) may alternatively or additionally be employed. Likewise in at least some embodiments of the invention where the root of a particular plant is indicated as being the preferred part of the plant (e.g. radix polygalae, radix astragali and radix angelicae sinensis) the rhizome (rhizoma) may alternatively or additionally be employed.

The Neuroaid2 components may be in their natural, herbal form (e.g. chopped into small pieces or ground to produce a powder) or in a more refined form (e.g. extracts), or combinations thereof. The starting material for the Neuroaid2 components may be the corresponding plants in fresh or dried form. The plants representing the various Neuroaid2 components may be processed individually or they may be combined and processed together. Optionally, the plants may be chopped into small pieces and, where necessary, dried. The dried ingredients may then optionally be ground to produce a powder. Plant material processed in this way may then be used in the various aspects of the invention.

Suitable methods for preparing herbal extracts will be known to those skilled in the art and include, for example, solid-liquid extraction, liquid-liquid extraction, supercritical fluid extraction, pressurized solvent extraction, microwave-assisted Extraction, subcritical water extraction, ultrasound-assisted extraction, and accelerated solvent extraction.

An extract according to the present invention may be prepared in a conventional manner, such as by combining plant material with one or more solvents under conditions suitable for preparing the extract. After the plant material and solvent have been in contact for a period of time suitable for forming the extract, the solvent and plant material is separated by a suitable method, such as filtering or centrifugation. The extract (i.e. the liquid comprising the solvent) may optionally be further processed, such as by concentrating or dehydrating the extract, combining the extract with further ingredients (e.g. diluents, other herbs / herbal extracts, TCM ingredients, preservatives etc.), or a mixture of the foregoing etc.

### Presentation of the Neuroaid2 Components

The various aspects of the invention relate to four particular Neuroaid2 components as previously defined and uses thereof. In the invention said Neuroaid2 components are provided in the form of a composition, such as a pharmaceutical composition. Thus, in the invention there is provided a composition (e.g. a pharmaceutical composition) which consists of as active ingredients said four particular Neuroaid2 components.

A pharmaceutical composition as described herein may optionally comprise a pharmaceutically acceptable additive, carrier or diluent and in addition, may include other active ingredients, pharmaceutical agents, carriers, adjuvants, etc. Examples of pharmaceutically acceptable additives include pharmaceutically acceptable excipients, buffers, adjuvants, stabilizers, diluents, fillers, buffers, stabilisers, preservatives, lubricants, or other pharmaceutically acceptable materials well known to those skilled in the art or as described herein. In at least some embodiments, a pharmaceutical composition as herein described comprises an excipient, and wherein said excipient is optionally dextrin or maldextrin.

In at least some embodiments a composition of the invention (e.g. a pharmaceutical composition) may be comprised within a kit.

It will be understood that the compositions as described herein may be administered in pure form or in an appropriate pharmaceutical composition. In general, pharmaceutical compositions may be prepared according to methods which are known to those of ordinary skill in the art. The compositions comprising the active components or ingredients disclosed herein may include a conventional pharmaceutical carrier or diluent and in addition may include other medicinal agents, pharmaceutical agents, carriers, adjuvants etc. Examples of suitable pharmaceutical carriers or diluents include phosphate buffered saline solutions, water, emulsions (such as oil/water emulsions), various types of wetting agents, sterile solutions etc. Examples of excipients which may be employed include, for example, sugars, starches, celluloses, gums, proteins, dextrin and maltodextrin. Various formulations are commonly known and are thoroughly discussed in the latest edition of Remington's Pharmaceutical Sciences (Maack Publishing, Easton PA).

The composition may, for example, be a solution, a suspension, liquid, chopped herbs, powder, a paste, aqueous, non-aqueous or any combination thereof.

The invention also provides a kit consisting of as active ingredients, the four particular Neuroaid 2 components (i.e. Chuanxiong, Angelica sinensis, Polygalae and Astragali).

In at least some embodiments, the kits of the invention may, in addition to the Neuroaid2 components, comprise instructions for use. The kits of the invention may be promoted, distributed, and/or sold as a unit for performing one of the aspects of the present invention.

In at least some embodiments, a kit of the invention comprises the Neuroaid2 components and an excipient (e.g. dextrin or maltodextrin).

The kits and compositions disclosed in the present specification may optionally be used as disclosed in the present specification.

In at least some embodiments, the kits and compositions disclosed in the present specification are used for neuroprotection, neuroconditioning, treating obesity, treating hypertension, or treating urinary incontinence.

### Administration of the Neuroaid2 Components

The Neuroaid2 components described herein may be administered by any suitable route, such as orally, parenterally, intravenously, subcutaneously, intradermally, intraperitoneally or topically, in liquid, semi-liquid or solid form and are formulated in a manner suitable for each route of administration. The term "administering" and variations of that term including "administer" and "administration", includes contacting, applying, delivering or providing a composition of the invention to an organism, or a surface by any appropriate means.

The Neuroaid2 components are to be administered in a therapeutically effective amount (either as a single dose or as part of a series of doses). By an "effective amount" or a "therapeutically effective amount" is meant the amount administered is physiologically significant. An agent is physiologically significant if it is present in an amount that results in a detectable change in the physiology of a recipient patient such that beneficial or desired results are achieved (e.g. to prevent, alleviate, or ameliorate one or more symptoms of a disease or medical condition, and/or to prolong the survival of the subject being treated).

The exact amount required will vary from subject to subject depending on factors such as the species being treated, the age, weight and general health of the subject, the condition being treated and the severity of the condition, the mode of administration, whether the treatment is prophylactic or to treat an existing condition, the gender of the subject, diet, time and frequency of administration, drug combination(s), and tolerance/response to therapy and so forth. Thus, it is not possible to specify an exact "effective amount". The effective dose for a given situation can be determined by routine experimentation and is within the judgement of the skilled person. For example, in order to formulate a range of dosage values, cell culture assays and animal studies can be used.

For illustration only, MLC 901 is typically administered in the form of 2 capsules, 3 times per day.

NeuroAid is typically administered orally (per os) 3 times each day and four 0.4g capsules each time. The duration of treatment is typically 3 months/3 courses, adaptable with regard to the patient's condition. This dosage is suitable for stroke treatment. For other diseases, the treatment can last longer. For patients with swallowing difficulties, capsules may be opened and powder diluted in water that can be drunk as such or injected via a gastric tube. Hence, a daily dose of about 4.8g is envisaged. In one embodiment, the patient's daily dose is about 1g to 8g; 2g to 8g; 3g to 7g; 4g to 6g; 4.25g to 5.75g; 4.5g to 5.25g; 4.5g to 5g; 4.6g to 4.10g; or 4.7g to 4.9g. A "daily dose" can be a single tablet or capsule etc. or multiple tablets or capsules etc. to be taken on a given day. However, it is to be understood that the dosages may be varied depending upon the requirement of the patients and the severity of the condition being treated etc.

Each course of NeuroAid treatment can last about 4 weeks. Typically 3 courses are administered, most commonly back to back. No therapeutic window is required but additional courses can be added even after a few days of treatment cessation. Hence, NeuroAid treatment can last about 12 weeks. The treatment course of NeuroAid can be about 4 to 24 weeks; 7 to 16 weeks; 9 to 15 weeks; 10 to 14 weeks; or 11 to 13 weeks.

### EXAMPLES

### EXAMPLE 1 (not forming part of the present invention)

NeuroAid (MLC601 and MLC901), a Traditional Medicine used in China for patients after stroke has been reported in preclinicals models of ischemia to induce neuroprotection and neuroplasticity. This work shows the effects of MLC901 on an *in vitro* model of oxygen glucose deprivation (OGD). MLC901 prevents neuronal death induced by 2 hours OGD and decreases the exaggerated Ca²⁺ entry induced by glutamate in cortical neurons exposed to 2 hours OGD. The neuroprotective effect of MLC901 is associated with a large hyperpolarization of ~ 20 mV which is antagonized by glibenclamide, the specific inhibitor of K_{ATP} channels. In addition MLC901 strengthens the activation of K_{ATP} channels. MLC901 has been directly shown to act as an activator of K_{ATP} channels as potent as the classical K_{ATP} channels opener. The capacity of MLC901 to produce a large hyperpolarization, particularly in neurons that have suffered from energy deprivation probably plays an important role in the neuroprotective effects of this TCM that comes in addition to its previously demonstrated neuroregenerative properties.

### MATERIALS AND METHODS

### Cell culture

### Cortical embryonic mouse neurons

### Primary culture of cortical neurons

Time-pregnant (E14) C57B16/J mice were anesthetized with isopentane followed by cervical dislocation as previously described (Heurteaux et al., 2010). Fetuses were removed and placed in cold HBSS⁺ solution. Cerebral cortices were dissected in cold HBSS⁺ solution and the meninges were removed. Cortical samples were cut in small pieces and were gently triturated with a fire-polished glass Pasteur pipette in 8 ml HBSS⁺ solution. The mix was filtered (40 µm filter) and centrifuged at 800 rpm for 8 min. The supernatant was removed and the pellet was dissolved in 2 ml culture medium. Cells were plated on poly-D-lysine (Sigma-Aldrich Chimie, St Quentin Fallavier, France)-coated 12 well (24 mm diameter) plates with glass coverslips (12 mm diameter) (CML, Nemours, France) at a density of 1 x 10⁶ cells/well. Cultures were maintained at 37°C in a humidified 95% air / 5% CO₂ atmosphere incubator in Neurobasal supplemented with B27, Glutamax, 100 units/ml penicillin, and 100g/ml streptomycin and used for experiments up to 12 days. Glial growth was suppressed by addition of 5-Fluoro-2-deoxyuridine (2 µM) and Uridine (2 µM) during the second day of culture.

### INS-R9 cell culture

INS-R9 cells were maintained in RPMI medium (Coppola et al., 2008) supplemented with 5% fetal calf serum, ImM sodium pyruvate, 2mM glutamate, 50M 2-mercaptoethanol, 100 units/ml penicillin, and 100g/ml streptomycin in an atmosphere of 95 % air / 5 % CO₂.

### Oxygen Glucose Deprivation (OGD) model

OGD experiments were performed on primary mouse cortical neurons seeded at a density of 1,000,000 cells/35-mm dish after 4 days of culture (Goldberg and Choi, 1993). After three washes with deoxygenated glucose-free Earl's balanced salt solution (BSS), cells were maintained in the same BSS medium. Composition of BSS solution was (in mM): 140 NaCl, 5.4 KCl, 1.2 CaCl₂, 0.9 MgCl₂, 0.44 KH₂PO₄, 4.17 NaHCO₃ and 0.34 Na₂HPO₄. Prior to use, BSS was equilibrated with the anaerobic gas mixture (1.2% O₂) by bubbling for 15 min, adjusted to pH 7.4 if necessary, and heated to 37°C. Then, cells were placed in humidified incubator at 37°C in anaerobic conditions (1.2% O₂) for two hours. Cortical neurons were treated with saline or MLC901 (1 µg/mL). Treatments were applied for two hours before OGD (pre-OGD condition), during OGD (OGD condition) or for two hours after OGD (post-OGD condition).

### Electrophysiology

### Whole cell current recordings

All electrophysiological experiments were done on INS-R9 transfected cells seeded at a density of 20,000 cells/35-mm dish. Transfected cells were used 48-72 h after transfection. All electrophysiological recordings were performed in whole cell configuration of the patch clamp technique (Hamill et al., 1981). Each current was evaluated by using a RK 400 patch clamp amplifier (Axon Instrument, USA), low-pass filtered at 3 kHz and digitized at 10 kHz using a 12-bit analog-to-digital converter digidata (1322 series, Axon Instrument, USA). All current amplitudes are expressed in current densities. Results are expressed as mean and standard error of the mean (SEM). Patch clamp pipettes were pulled using vertical puller (PC-10, Narishige) from borosilicate glass capillaries and had a resistance of 3-5 MΩ. For K⁺ currents the bath solution contained (in mM) 150 NaCl, 5 KCl, 3 MgCl₂, 1 CaCl₂ and 10 HEPES adjusted to pH 7.4 with NaOH. The pipette solution contained (in mM) 155 KCl, 3 MgCl₂, 5 EGTA and 10 HEPES adjusted to pH 7.2 with KOH. For Na⁺ currents, the bath solution contained (in mM) 150 NaCl, 2 KCl, 1 MgCl₂, 1.5 CaCl₂, 10 glucose and 10 HEPES adjusted to pH 7.4 with NaOH. The pipette solution contained (in mM) 135 CsCl, 2 MgCl₂, 2.5 Na₂-ATP, 5 EGTA, 2.1 CaCl₂, and 10 HEPES adjusted to pH 7.2 with CsOH. All experiments were performed at room temperature (21-22°c). Stimulation protocols and data acquisition were carried out using a microcomputer (Dell Pentium) witch used a commercial software and hardware (pClamp 8.2). K⁺ Currents were recorded by voltage clamp steps to membrane potentials of - 140 to + 80 mV for neurons or - 100 to + 60 mV for transfected cell lines in 20 mV steps applied from a holding potential of - 80 mV. Duration of depolarization pulses were 0.825 ms, and the pulse cycling rate was 5 s. Current amplitudes were evaluated at the end of the stimulation pulses. Na⁺ currents were recorded by voltage clamp steps to membrane potentials of - 80 to + 50 mV in 5 mV steps applied from a holding potential of - 100 mV. The duration of a depolarization pulse was 100 ms, and the pulse cycling rate was 2 s. Current amplitudes were evaluated at the peak of the stimulation pulses. To isolate the native Na⁺ currents, TEA was used in the extracellular solution to block K⁺ channels. The presence of cadmium in the extracellular solution allowed the blockade of calcium currents in these neurons. Cells were continuously superfused with a microperfusion system. For INS-R9 cells, currents were recorded in control conditions, then in the presence of MLC901 (1 µg/mL), MLC901 + Pinacidil (10 µM) and MLC901 + Pinacidil + Glibenclamide (10 µM). Control, Pinacil, Pinacidil + MLC901 and Pinacidil + MLC901 + Glibenclamide (10 µM) sequence perfusion was also performed.

### Oocyte recordings

Defolliculated Xenopus oocytes were injected with 100 nL of cRNA at 0.02-0.4 µg/µL for Kir 6.2 and SUR 1 expression, and recorded 2-4 days later.

For electrophysiology, single oocytes were placed in a 0.3-mL perfusion chamber and impaled with two standard microelectrodes (1-2.5 MΩ resistance) filled with 3 M KCl and voltage clamped with a Dagan CA-1 amplifier, in symmetrical potassium solution containing (in mM) (90 KCl, 1.8 CaCl₂, 1 mM MgCl₂, 5 Hepes, pH 7.4 with KOH). Stimulation of the preparation, data acquisition, and analysis were performed using pClamp software (Axon Instruments). Currents were recorded in this potassium symmetrical control condition. K_{ATP} channel activity was evaluated by intracellular ATP depletion by 3 mM of sodium azide. MLC901 (1 µg/mL) and Pinacidil (10 µM) were tested before and after oocyte ATP depletion. In all experiments, inhibition by Glibenclamide (10 µM) of recorded currents was evaluated.

### Statistical Analyses.

Data were expressed as mean ± S.E.M. Statistical analysis of differences between groups was performed by using unpaired t test or ANOVA. Where F ratios were significant, statistical analyses were extended and post-hoc comparisons made by using Tukey's test multiple comparison tests. In all analyses, the level of significance was set at P<0.05.

### RESULTS

### MLC901 protects cortical neurons against death associated with oxygen glucose deprivation

The effects of MLC901 were studied in an *in vitro* model of ischemia in which cortical neurons were exposed to a glucose and oxygen deprivation. Hoechst staining of nuclei of living neurons was used to assess OGD-induced neuronal death in cultured neurons. Cortical neurons were first treated with saline or MLC901 (1 µg/mL). The dose of MLC901 used was selected based on our previous study, where the application of 1 µg/mL MLC901 induced the best protection against cell death on cortical neurons in culture (Heurteaux et al., 2010). During OGD challenge, cells were exposed to three treatment conditions: MLC901 was applied 1/ for two hours before OGD (pre-OGD condition), 2/ during 2 hours OGD (OGD condition) and 3/ for two hours after OGD (post-OGD condition). We compared the protective effects of MLC901 against OGD-induced neurodegeneration of cortical cells. Severe OGD induced a dramatic decrease in the number of living Hoechst-positive cells. MLC601 treatment before, during or after OGD resulted in a significant increase in neuronal viability as compared to respective controls (****P*< 0.001) (n = 12 35 mm dishes per group) (Figure 1). MLC901 application after OGD gave the better protection (Figure 1). An application of a specific inhibitor of K_{ATP} channels, glibenclamide (Fosset et al., 1988, Bernardi et al., 1988) inhibited the protection induced by MLC901, suggesting an involvement of K_{ATP} channels in the protective effect of MLC901 against OGD damage (Figure 1).

### The neuroprotective effect of MLC901 may be linked to K_{ATP} channel opening

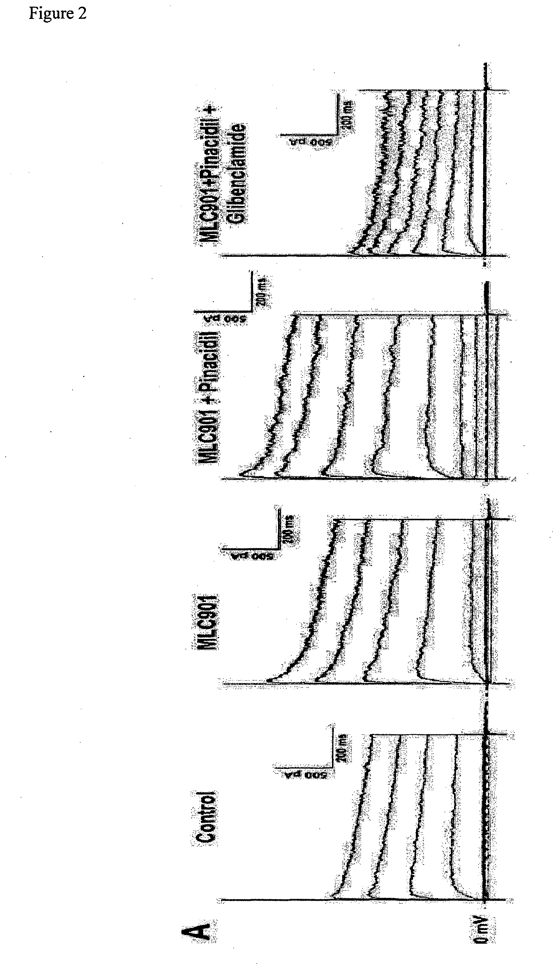

We carried out experiments on rat INS-R9 insulinoma cells that are known to express glibenclamide-sensitive K_{ATP} channels that are essential for insulin secretion. K_{ATP} channels in INS-R9 cells were significantly activated by MLC901 (1 µg/mL) and we observed an additive effect with the application of 10 µM pinacidil, a well known activator of K_{ATP} channels (Edwards and Weston, 1990; Mannhold, 2004; Quast, 1992) (Figure 2). Glibenclamide (10 µM) inhibited the K_{ATP} current activated by MLC901 + Pinacidil (Figure 2). The results obtained in INS-R9 cells confirm that the electrophysiological effects of MLC901 are, at least in part, mediated by K_{ATP} channels.

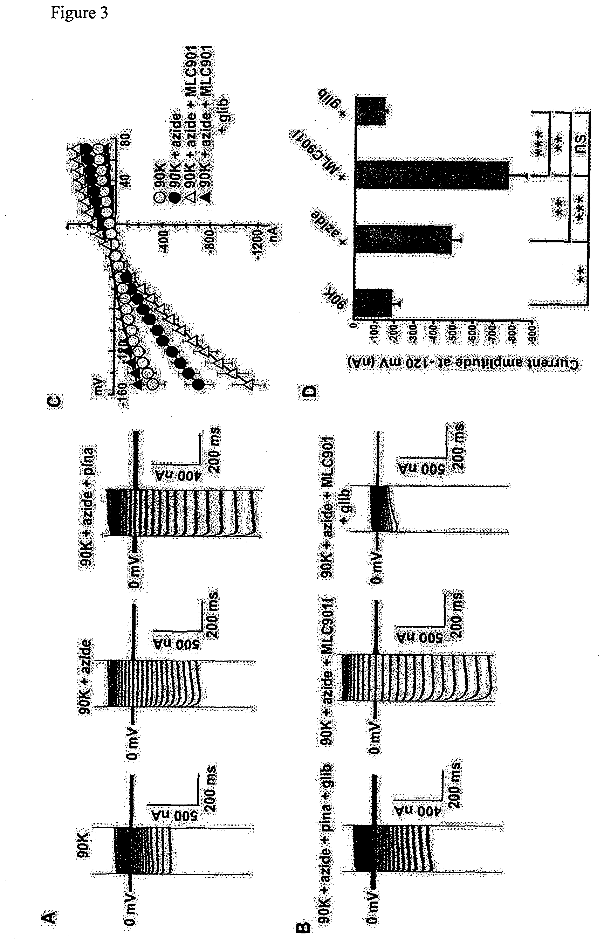

The two subunits that constitute the neuronal K_{ATP} channel are SUR₁ and Kir_{6.2} (Inagaki et al., 1995). Xenopus oocyte expression of SUR₁ and Kir_{6.2}, led to generation of large inwardly rectifying currents in response to application of sodium azide (3 mM) to decrease intracellular ATP and increase the ADP/ATP ratio (Ashcroft and Ashcroft, 1990) (Figure 3A). The amplitude of the azide-induced, glibenclamide-sensitive K_{ATP} channel current was amplified by application of pinacidil (10 µM) and as expected inhibited by glibenclamide (10 µM) (Figure 3A). We then performed the same type of experiments by using MLC901. Clearly, MLC901, like pinacidil activated K_{ATP} channels revealed by the azide treatment (Figure 3B-C-D). Twelve minutes after azide application, the mean current amplitude at -120 mV was - 480 ± 57 nA before and -770 ± 67 after application of MLC901. This MLC901-induced activation like that which was produced by pinacidil disappeared in the presence of glibenclamide (Figure 3B-C-D). MLC901 behaves similarly to pinacidil by activating the K_{ATP} channel.

### DISCUSSION.

These results described here demonstrate that MLC901 protects cortical neurons against death in an *in vitro* model of ischemia. The model of oxygen glucose deprivation mimics cell death processes observed in the salvageable (penumbral) regions of the ischemic brain *in vivo.* MLC901 significantly prevents neuronal cells from death. This neuroprotective effect is blocked by glibenclamide, the specific inhibitor of K_{ATP} channels.

The activating effect of MLC901 on K_{ATP} channels has been proved by electrophysiological experiments on ovocytes expressing recombinant channels and insulinoma cells. Insulinoma cells naturally have K_{ATP} channels, essential to couple changes of extracellular glucose levels to insulin secretion (Lazdunski, 1996). MLC901 induces a large hyperpolarisation in these both types of cells. This large hyperpolarization produced by MLC901 is expected to strongly protect neurons against death. MLC901 behaved like pinacidil, a classical K_{ATP} channel opener (Edwards and Weston, 1990; Mannhold, 2004, Heurteaux et al., 1995), and the stimulating effects of both MLC901 and pinacidil were abolished by glibenclamide.

The activating effect of MLC901 on K_{ATP} channels are observed at therapeutic concentrations used in rodent models of ischemia (Heurteaux et al., 2010; Quintard et al., 2011). Therefore, in addition to its neuroregenerative properties (Heurteaux et al., 2010), the cocktail of active molecules present in this TCM seems to act via K_{ATP} channels for at least a part of its properties against cerebral ischemia.

### EXAMPLE 2

### Oocyte recordings

For Kir 6.2 and SUR 1 expression defolliculated Xenopus oocytes were injected with 100 nL of cRNA at 0.02-0.4 µg/µL, and currents were recorded after 2-4 days. Each oocyte was placed in a 0.3-mL perfusion chamber and impaled with two standard microelectrodes (1-2.5 MΩ resistance) filled with 3 M KCl and voltage clamped with a Dagan CA-1 amplifier, in symmetrical potassium solution containing (in mM) (90 KCl, 1.8 CaCl₂, 1 mM MgCl₂, 5 Hepes, pH 7.4 with KOH). Stimulation of the egg, data acquisition, and analysis were performed using pClamp software (Axon Instruments). Currents were recorded in the potassium symmetrical control condition. K_{ATP} channel activity was evaluated by intracellular ATP depletion by 3 mM of sodium azide.

### Perfusion protocol:

Current was first recorded in control condition (symmetrical potassium solution). Then, channel activity was enhanced by 6 minutes of 3 mM sodium azide perfusion which was maintened during the entire experiment. MLC901 or other batch products were perfused 6 minutes after sodium azide perfusion. In all experiments, inhibition of recorded currents by Glibenclamide (10 µM) was evaluated.

### RESULTS

The results are shown in Figures 4 and 5. Both NA4A (combination of Astragalus + Chaunxiong + Sinensis + Polygala) falling within the scope of the present invention and NA4B (combination of Astragalus + Chaunxiong + Sinensis + Salvia) not forming part of the invention activated the K(ATP) channel preactivated by azide. The increase of the current induced by the opening of the channel is inhibited by the addition of glibenclamide. 90K is the current of the cell not treated

### EXAMPLE 3 (not forming part of the present invention)

The in vitro effects of both samples (Neuroaid2 and "NA3" (chuanxiong, sinensis, astragalus)) on cloned Kir6.2/SUR2A potassium channels (expressed by the human KCNJ11 and SUR2A genes and coexpressed in HEK293 cells) were evaluated at room temperature using the QPatch HT® (Sophion Bioscience A/S, Denmark), an automatic parallel patch clamp system. The samples were evaluated at 10 µg/ml with each concentration tested in two or more cells (n ≥ 2). The channels were activated with an 8 minute exposure to 10 µM pinacidil and the duration of exposure to each test article concentration was 5 minutes.

### RESULTS

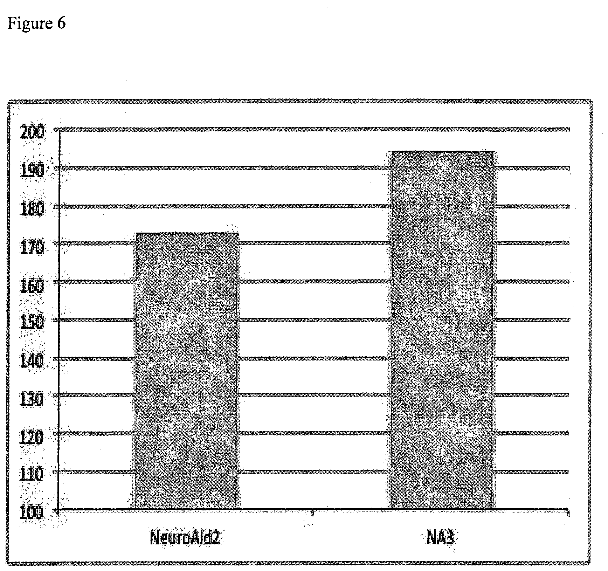

The results are shown in Figure 6. Both samples NeuroAid2 and NA3 are able to activate the channel, data are expressed in percentage of activation of the channel.

The activation of the channel by both samples is inhibited by an exposure of Glybenclamide.

### REFERENCES

Amoroso, S., Schmid-Antomarchi, H., Fosset, M., Lazdunski, M., 1990. Glucose, sulfonylureas, and neurotransmitter release: role of ATP-sensitive K+ channels. Science 247, 852-854.
Ashcroft, S. J., Ashcroft, F. M., 1990. Properties and functions of ATP-sensitive K-channels. Cell Signal 2, 197-214.
Bernardi, H., Fosset, M., Lazdunski, M., 1988. Characterization, purification, and affinity labeling of the brain [3H]glibenclamide-binding protein, a putative neuronal ATP-regulated K+ channel. Proc Natl Acad Sci U S A 85, 9816-9820.
Blondeau, N., Nguemeni, C., Debruyne, D. N., Piens, M., Wu, X., Pan, H., Hu, X., Gandin, C., Lipsky, R. H., Plumier, J. C., Marini, A. M., Heurteaux, C., 2009. Subchronic alpha-linolenic acid treatment enhances brain plasticity and exerts an antidepressant effect: a versatile potential therapy for stroke. Neuropsychopharmacology 34, 2548-2559.
Blondeau, N., Plamondon, H., Richelme, C., Heurteaux, C., Lazdunski, M., 2000. K(ATP) channel openers, adenosine agonists and epileptic preconditioning are stress signals inducing hippocampal neuroprotection. Neuroscience 100, 465-474.
Blondeau, N., Widmann, C., Lazdunski, M., Heurteaux, C., 2002. Polyunsaturated fatty acids induce ischemic and epileptic tolerance. Neuroscience 109, 231-241.
Catterall, W. A., 2000. From ionic currents to molecular mechanisms: the structure and function of voltage-gated sodium channels. Neuron 26, 13-25.
Chen, C., Venketasubramanian, N., Gan, R. N., Lambert, C., Picard, D., Chan, B. P., Chan, E., Bousser, M. G., Xuemin, S., 2009. Danqi Piantang Jiaonang (DJ), a traditional Chinese medicine, in poststroke recovery. Stroke 40, 859-863.
Choi, D. W., 1988. Glutamate neurotoxicity and diseases of the nervous system. Neuron 1, 623-634.
Coppola, T., Beraud-Dufour, S., Antoine, A., Vincent, J. P., Mazella, J., 2008. Neurotensin protects pancreatic beta cells from apoptosis. Int J Biochem Cell Biol 40, 2296-2302. Dirnagl, U., Iadecola, C., Moskowitz, M. A., 1999. Pathobiology of ischaemic stroke: an integrated view. Trends Neurosci 22, 391-397.
Edwards, G., Weston, A. H., 1990. Structure-activity relationships of K+ channel openers. Trends Pharmacol Sci 11, 417-422.
Enyedi, P., Czirjak, G., 2010. Molecular background of leak K+ currents: two-pore domain potassium channels. Physiol Rev 90, 559-605.
Fosset, M., De Weille, J. R., Green, R. D., Schmid-Antomarchi, H., Lazdunski, M., 1988. Antidiabetic sulfonylureas control action potential properties in heart cells via high affinity receptors that are linked to ATP-dependent K+ channels. J Biol Chem 263, 7933-7936.
Gan, R., Lambert, C., Lianting, J., Chan, E. S., Venketasubramanian, N., Chen, C., Chan, B. P., Samama, M. M., Bousser, M. G., 2008. Danqi Piantan Jiaonang does not modify hemostasis, hematology, and biochemistry in normal subjects and stroke patients. Cerebrovasc Dis 25, 450-456.
Goldberg, M. P., Choi, D. W., 1993. Combined oxygen and glucose deprivation in cortical cell culture: calcium-dependent and calcium-independent mechanisms of neuronal injury. J Neurosci 13, 3510-3524.
Gribkoff, V. K., Winquist, R. J., 2005. Voltage-gated cation channel modulators for the treatment of stroke. Expert Opin Investig Drugs 14, 579-592.
Hamill, O. P., Marty, A., Neher, E., Sakmann, B., Sigworth, F. J., 1981. Improved patch-clamp techniques for high-resolution current recording from cells and cell-free membrane patches. Pflugers Arch 391, 85-100.
Heurteaux, C., Bertaina, V., Widmann, C., Lazdunski, M., 1993. K+ channel openers prevent global ischemia-induced expression of c-fos, c-jun, heat shock protein, and amyloid beta-protein precursor genes and neuronal death in rat hippocampus. Proc Natl Acad Sci USA 90, 9431-9435.
Heurteaux, C., Gandin, C., Borsotto, M., Widmann, C., Brau, F., Lhuillier, M., Onteniente, B., Lazdunski, M., 2010. Neuroprotective and neuroproliferative activities of NeuroAid (MLC601, MLC901), a Chinese medicine, in vitro and in vivo. Neuropharmacology 58, 987-1001.
Heurteaux, C., Guy, N., Laigle, C., Blondeau, N., Duprat, F., Mazzuca, M., Lang-Lazdunski, L., Widmann, C., Zanzouri, M., Romey, G., Lazdunski, M., 2004. TREK-1, a K(+) channel involved in neuroprotection and general anesthesia. Embo J 23, 2684-2695.
Heurteaux, C., Laigle, C., Blondeau, N., Jarretou, G., Lazdunski, M., 2006. Alpha-linolenic acid and riluzole treatment confer cerebral protection and improve survival after focal brain ischemia. Neuroscience 137, 241-251.
Heurteaux, C., Lauritzen, I., Widmann, C., Lazdunski, M., 1995. Essential role of adenosine, adenosine A1 receptors, and ATP-sensitive K+ channels in cerebral ischemic preconditioning. Proc Natl Acad Sci U S A 92, 4666-4670.
Inagaki, N., Gonoi, T., Clement, J. P. t., Namba, N., Inazawa, J., Gonzalez, G., Aguilar-Bryan, L., Seino, S., Bryan, J., 1995. Reconstitution of IKATP: an inward rectifier subunit plus the sulfonylurea receptor. Science 270, 1166-1170.
Lauritzen, I., Blondeau, N., Heurteaux, C., Widmann, C., Romey, G., Lazdunski, M., 2000. Polyunsaturated fatty acids are potent neuroprotectors. Embo J 19, 1784-1793.
Lauritzen, I., De Weille, J. R., Lazdunski, M., 1997. The potassium channel opener (-)-cromakalim prevents glutamate-induced cell death in hippocampal neurons. J Neurochem 69, 1570-1579.
Lazdunski, M., 1996. Ion channel effects of antidiabetic sulfonylureas. Horm Metab Res 28, 488-495.
Lee, J. M., Grabb, M. C., Zipfel, G. J., Choi, D. W., 2000. Brain tissue responses to ischemia. J Clin Invest 106, 723-731.
Legos, J. J., Barone, F. C., 2003. Update on pharmacological strategies for stroke: prevention, acute intervention and regeneration. Curr Opin Investig Drugs 4, 847-858.
Lesage, F., 2003. Pharmacology of neuronal background potassium channels. Neuropharmacology 44, 1-7.
Lesage, F., Lazdunski, M., 2000. Molecular and functional properties of two pore domain potassium channels. Am. J. Physiol. 279, 793-801.
Leybaert, L., De Ley, G., de Hemptinne, A., 1993. Effects of flunarizine on induced calcium transients as measured in fura-2-loaded neurons of the rat dorsal root ganglion. Naunyn Schmiedebergs Arch Pharmacol 348, 269-274.
Liss, B., Roeper, J., 2001. Molecular physiology of neuronal KATP channels. Mol Membr Biol 18, 117-127.
Mannhold, R., 2004. KATP channel openers: structure-activity relationships and therapeutic potential. Med Res Rev 24, 213-266.
Mazighi, M., Amarenco, P., 2011. Reperfusion therapy in acute cerebrovascular syndrome. Curr Opin Neurol 24, 59-62.
Mourre, C., Ben Ari, Y., Bernardi, H., Fosset, M., Lazdunski, M., 1989. Antidiabetic sulfonylureas: localization of binding sites in the brain and effects on the hyperpolarization induced by anoxia in hippocampal slices. Brain Res 486, 159-164.
Obrenovitch, T. P., 1997. Sodium and potassium channel modulators: their role in neuroprotection. Int Rev Neurobiol 40, 109-135.
Obrenovitch, T. P., 2008. Molecular physiology of preconditioning-induced brain tolerance to ischemia. Physiol Rev 88, 211-247.
Plamondon, H., Blondeau, N., Heurteaux, C., Lazdunski, M., 1999. Mutually protective actions of kainic acid epileptic preconditioning and sublethal global ischemia on hippocampal neuronal death: involvement of adenosine A1 receptors and KATP channels. J Cereb Blood Flow Metab 19, 1296-1308.
Quast, U., 1992. Potassium channel openers: pharmacological and clinical aspects. Fundam Clin Pharmacol 6, 279-293.
Quintard, H., Borsotto, M., Veyssiere, J., Gandin, C., Labbal, F., Widmann, C., Lazdunski, M., Heurteaux, C., 2011. MLC901, a traditional Chinese medicine protects the brain against global ischemia. Neuropharmacology 61, 622-631.
Shahripour, R. B., Shamsaei, G., Pakdaman, H., Majdinasab, N., Nejad, E. M., Sajedi, S. A., Norouzi, M., Hemmati, A., Manouchehri, R. H., Shiravi, A., 2011. The effect of NeuroAid (MLC601) on cerebral blood flow velocity in subjects' post brain infarct in the middle cerebral artery territory. Eur J Intern Med 22, 509-513.
Venketasubramanian, N., Chen, C. L., Gan, R. N., Chan, B. P., Chang, H. M., Tan, S. B., Picard, D., Navarro, J. C., Baroque, A. C., 2nd, Poungvarin, N., Donnan, G. A., Bousser, M. G., 2009. A double-blind, placebo-controlled, randomized, multicenter study to investigate CHInese Medicine Neuroaid Efficacy on Stroke recovery (CHIMES Study). Int J Stroke 4, 54-60.
Young, S. H., Zhao, Y., Koh, A., Singh, R., Chan, B. P., Chang, H. M., Venketasubramanian, N., Chen, C., 2010. Safety profile of MLC601 (Neuroaid) in acute ischemic stroke patients: A Singaporean substudy of the Chinese medicine Neuroaid efficacy on stroke recovery study. Cerebrovasc Dis. 30, 1-6.

## Claims

1. A composition or kit for use in a method of providing neuroprotection, treating stroke, a neurological disorder, ischemia, or providing neuroconditioning to provide tolerance to the brain against an ischaemic, epileptic or other injurious event, wherein the composition or kit consists of as active ingredients the following four components:
i. Polygalae (thinleaf milkwort, Polygala tenuifolia Willd., Polygala sibirica L. or Yuanzhi), preferably the root thereof (i.e. radix polygalae);
ii. Astragali (Membranous Milkvetch or Huang Qi), preferably the root (i.e. radix astragali);
iii. Chuanxiong, preferably the rhizome thereof (i.e. rhizoma chuanxiong); and
iv. Angelicae sinensis (Chinese Angelica or DanGui), preferably the root (i.e. radix angelicae sinensis).

2. The composition for use according to claim 1, wherein the composition is a pharmaceutical composition which comprises a pharmaceutically acceptable additive, carrier or diluent.

3. The composition or kit for use according to claim 1 or claim 2, wherein the neuroprotection is against stroke or a degenerative disorder.

4. The composition or kit for use according to any one of claims 1 to 3, wherein the stroke is cerebral stroke.

5. The composition or kit for use according to any one of claims 1 to 3, wherein the stroke is cardiovascular disease (heart stroke mainly due to coronary artery stroke).

6. The composition or kit for use according to claim 1 or claim 2, wherein the neurological disorder is a neurodegenerative disease or a traumatic injury to the brain.

7. A composition or kit consisting of as active ingredients the following four components:
i. Polygalae (thinleaf milkwort, Polygala tenuifolia Willd., Polygala sibirica L. or Yuanzhi), preferably the root thereof (i.e. radix polygalae);
ii. Astragali (Membranous Milkvetch or Huang Qi), preferably the root (i.e. radix astragali);
iii. Chuanxiong, preferably the rhizome thereof (i.e. rhizoma chuanxiong); and
iv. Angelicae sinensis (Chinese Angelica or DanGui), preferably the root (i.e. radix angelicae sinensis).

8. The composition or kit according to claim 7 comprising a pharmaceutically acceptable additive, carrier or diluent.

## Patentansprüche

1. Zusammensetzung oder Kit zur Verwendung in einem Verfahren zur Bereitstellung eines neurologischen Schutzes, zur Behandlung eines Schlaganfalls, einer neurologischen Störung, von Ischämie oder zur Bereitstellung einer Neurokonditionierung zur Bereitstellung einer Toleranz für das Gehirn gegenüber einem ischämischen, epileptischen oder einem anderen verletzenden Ereignis, wobei die Zusammensetzung oder das Kit aus den folgenden vier Komponenten als Wirkstoffe besteht:
i. Polygalae (dünnblättrige Kreuzblume, Polygala tenuifolia Willd., Polygala sibirica L. oder Yuanzhi), vorzugsweise deren Wurzel (d.h., Radix polygalae);
ii. Astragali (mongolischer ("membranous") Tragant oder Huang Qi), vorzugsweise dessen Wurzel (d.h., Radix astragali);
iii. Chuanxiong, vorzugsweise dessen Rhizom (d.h., Rhizoma chuanxiong); und
iv. Angelica sinensis (chinesische Aralie oder DanGui), vorzugsweise deren Wurzel (d.h., Radix angelicae sinensis).

2. Zusammensetzung zur Verwendung nach Anspruch 1, wobei die Zusammensetzung eine pharmazeutische Zusammensetzung ist, die einen pharmazeutisch verträglichen Zusatz, einen pharmazeutisch verträglichen Träger oder ein pharmazeutisch verträgliches Verdünnungsmittel umfasst.

3. Zusammensetzung oder Kit zur Verwendung nach Anspruch 1 oder Anspruch 2, wobei der neurologische Schutz gegen einen Schlaganfall oder eine degenerative Störung gerichtet ist.

4. Zusammensetzung oder Kit zur Verwendung nach einem der Ansprüche 1 bis 3, wobei der Schlaganfall ein Gehirnschlag ist.

5. Zusammensetzung oder Kit zur Verwendung nach einem der Ansprüche 1 bis 3, wobei der Schlaganfall eine kardiovaskuläre Erkrankung (Herzschlag vorwiegend aufgrund eines Koronararterienschlaganfalls) ist.

6. Zusammensetzung oder Kit zur Verwendung nach Anspruch 1 oder Anspruch 2, wobei die neurologische Störung eine neurodegenerative Erkrankung oder eine traumatische Verletzung des Gehirns ist.

7. Zusammensetzung oder Kit, die oder das aus den folgenden vier Komponenten als Wirkstoffe besteht:
i. Polygalae (dünnblättrige Kreuzblume, Polygala tenuifolia Willd., Polygala sibirica L. oder Yuanzhi), vorzugsweise deren Wurzel (d.h., Radix polygalae);
ii. Astragali (mongolischer ("membranous") Tragant oder Huang Qi), vorzugsweise dessen Wurzel (d.h., Radix astragali);
iii. Chuanxiong, vorzugsweise dessen Rhizom (d.h., Rhizoma chuanxiong); und
iv. Angelica sinensis (chinesische Aralie oder DanGui), vorzugsweise deren Wurzel (d.h., Radix angelicae sinensis).

8. Zusammensetzung oder Kit nach Anspruch 7, die oder das einen pharmazeutisch verträglichen Zusatz, einen pharmazeutisch verträglichen Träger oder ein pharmazeutisch verträgliches Verdünnungsmittel umfasst.

## Revendications

1. Composition ou kit pour utilisation dans un procédé visant à conférer une neuroprotection, à traiter un accident vasculaire, un trouble neurologique, une ischémie, ou à conférer un neuroconditionnement pour conférer une tolérance au cerveau contre un événement ischémique, épileptique ou autre événement préjudiciable, dans lequel la composition ou le kit consiste, en tant qu'ingrédients actifs, en les quatre composants suivants :
i. Polygalae (millepertuis à feuilles minces, Polygala tenuifolia Willd., Polygala sibirica L. ou Yuanzhi), de préférence sa racine (i.e. radix polygalae) ;
ii. Astragali (Astragale ou Huang Qi), de préférence la racine (i.e. radix astragali) ;
iii. Chuanxiong, de préférence son rhizome (i.e. rhizoma chuanxiong) ; et
iv. Angelicae sinensis (Aralie de Chine ou DanGui), de préférence la racine (i.e. radix angelicae sinensis).

2. Composition pour utilisation selon la revendication 1, dans laquelle la composition est une composition pharmaceutique qui comprend un additif, support ou diluant pharmaceutiquement acceptable.

3. Composition ou kit pour utilisation selon la revendication 1 ou la revendication 2, dans laquelle la neuroprotection se fait contre un accident vasculaire ou un trouble dégénératif.

4. Composition ou kit pour utilisation selon l'une quelconque des revendications 1 à 3, dans laquelle l'accident vasculaire est un accident vasculaire cérébral.

5. Composition ou kit pour utilisation selon l'une quelconque des revendications 1 à 3, dans laquelle l'accident vasculaire est une maladie cardiovasculaire (accident vasculaire cardiaque principalement dû à un accident vasculaire de l'artère coronaire).

6. Composition ou kit pour utilisation selon la revendication 1 ou la revendication 2, dans laquelle le trouble neurologique est une maladie neurodégénérative ou une lésion traumatique causée au cerveau.

7. Composition ou kit consistant, en tant qu'ingrédients actifs, en les quatre composants suivants :
i. Polygalae (millepertuis à feuilles minces, Polygala tenuifolia Willd., Polygala sibirica L. ou Yuanzhi), de préférence sa racine (i.e. radix polygalae) ;
ii. Astragali (Astragale ou Huang Qi), de préférence la racine (i.e. radix astragali) ;
iii. Chuanxiong, de préférence son rhizome (i.e. rhizoma chuanxiong) ; et
iv. Angelicae sinensis (Aralie de Chine ou DanGui), de préférence la racine (i.e. radix angelicae sinensis).

8. Composition ou kit selon la revendication 7, comprenant un additif, support ou diluant pharmaceutiquement acceptable.
